# EUROPÄISCHE PATENTSCHRIFT

(11) **EP 2 121 701 B1**
(45) Veröffentlichungstag und Bekanntmachung des Hinweises auf die Patenterteilung: **06.11.2013**
(21) Anmeldenummer: 08706020.8
(22) Anmeldetag: 14.02.2008
(51) Int. Cl.: C07D 491/10, A61K 31/55, A61P 25/28

(54) **Verfahren zur Herstellung von hochreinem 4a,5,9,10,11,12-Hexahydro-6H-benzofuro[3a,3,2-ef][2]benzazepin sowie dessen Derivaten**
Process for the production of high-purity 4a,5,9,10,11,12-hexahydro-6H-benzofuro[3a,3,2-ef][2]benzazepine, and the derivatives thereof
Procédé de production de 4a,5,9,10,11,12-hexahydro-6H-benzofuro[3a,3,2-ef][2]benzazépine très pure et de dérivés de celle-ci

(30) Priorität: 22.02.2007 AT 2802007
(43) Veröffentlichungstag der Anmeldung: 25.11.2009
(73) Patentinhaber: Sanochemia Pharmazeutika AG, 1090 Wien (AT)
(72) Erfinder: WELZIG, Stefan, A-1030 Wien (AT); GERDENITSCH, Anton, A-7222 Rohrbach (AT); ROTHENBURGER, Jan, A-7064 Oslip (AT); KOLAR, Susanne, A-2491 Neufeld/Leitha (AT); SCHERLEITHNER, Alexandra, A-2491 Neufeld/Leitha (AT)
(74) Vertreter: Beer & Partner Patentanwälte KG
(86) Internationale Anmeldenummer: PCT/AT2008/000050
(87) Internationale Veröffentlichungsnummer: WO 2008/101266

(56) Entgegenhaltungen:
- WO-A-96/12692
- WO-A-97/11077
- SHIEH W-C ET AL: "ASYMMETRIC TRANSFORMATION OF EITHER ENANTIOMER OF NARWEDINE VIA TOTAL SPONTANEOUS RESOLUTION PROCESS A CONCISE SOLUTION TO THE SYNTHESIS OF (-)-GALANTHAMINE" JOURNAL OF ORGANIC CHEMISTRY, AMERICAN CHEMICAL SOCIETY. EASTON, Bd. 59, Nr. 18, 9. September 1994 (1994-09-09), Seiten 5463-5465, XP000562837 ISSN: 0022-3263
- CZOLLNER L ET AL: "New Kilogram-Synthesis of the Anti-Alzheimer Drug (-)-Galanthamine" TETRAHEDRON LETTERS, ELSEVIER, AMSTERDAM, Bd. 39, Nr. 15, 9. April 1998 (1998-04-09), Seiten 2087-2088, XP004110636 ISSN: 0040-4039

## Beschreibung

Die Erfindung betrifft Verfahren zum Herstellen von hochreinem 4a,5,9,10,11,12,-Hexahydro-6H-benzofuro[3a,3,2-ef][2]benzazepin sowie dessen Derivaten mit der allgemeinen Formel I und II 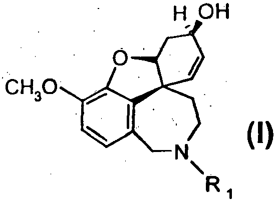 und 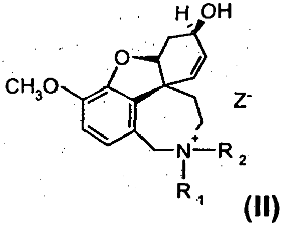 oder Salzen derselben, worin R₁ ausgewählt ist aus der Gruppe bestehend aus Wasserstoff, Hydroxy, Alkoxy, niedriges Alkyl, welches gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und worin R₂ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl und wobei Z⁻ ein Anion einer pharmazeutisch annehmbaren organischen Säure oder ein anorganisches Anion ist.

Galanthamin ist ein vorwiegend in Pflanzen vom Typus Amaryllidaceae vorkommendes Alkaloid mit hoher pharmakologischer Aktivität. Hervorzuheben ist insbesondere seine Wirkung als selektiver Acetylcholinesterase Inhibitor und die damit in Zusammenhang stehende Anwendung bei Behandlung neurodegenerativer Erkrankungen, wie die Alzheimer'sche Erkrankung. Die aus dem natürlich vorkommenden kaukasischen Schneeglöckchen Galanthus Woronoyi isolierten Mengen sind jedoch nicht ausreichend, um den Bedarf eines pharmazeutischen Rohstoffes abzudecken. Seit Ende der sechziger Jahres sind daher Galanthaminsynthesen bekannt, welche aber mitunter lange und unwirtschaftliche Reaktionswege mit schlechten Gesamtaüsbeuten zeigen.

Gemäß der WO-A-97/110777 soll durch gezielte Auswahl von Bromnarwedin als Ausgangsprodukt ein wirtschaftlicher Weg für die Galanthaminsynthese insofern geschaffen werden, als Bromnarwedin mit Palladium (II) Acetat unter Zusatz von Triphenylphosphin debromiert wird. Das erhaltene racemische Narwedin enthält allerdings etwa 700 - 800 ppM Palladium, welches auch nach mehrmaliger Behandlung mit Aktivkohle nicht abgetrennt werden kann. Auch bei weiteren Reaktionsschritten, wie die Reduktion von racemischem Narwedin, welche gemäß der WO-A-96/12692 der Anmelderin beschrieben wird, wird Palladium trotz mehrmaliger Aufarbeitung weiter im Reaktionsendprodukt nachgewiesen.

Galanthamin bzw. Galanthaminderivate, welche Palladium in einem Ausmaß von 700 - 800 ppM aufweisen, sind jedoch für die Herstellung von Arzneimitteln, wie Mittel zur Behandlung der Alzheimer'schen Erkrankung nicht geeignet, da im Organismus, bedingt durch die Palladiumreste, unerwünschte Nebenwirkungen auftreten können. Demgemäß sind Grenzwerte mit <5 ppm für die orale Applikation von Arzneimitteln normiert, siehe "Note for Guidance on specification limits for residues of metal catalysts" CPMP/SWP/QWP/4446/00.

Der Erfindung liegt daher die Aufgabe zugrunde, Verfahren der eingangs genannten Art anzugeben, mit welchen die vorgenannten, normierten Grenzwerte eingehalten werden können.

Erfindungsgemäß wird ein Verfahren zur Herstellung der eingangs genannten Verbindungen mit der allgemeinen Formel (I) bzw. (II) vorgeschlagen, wobei man in einem Reaktionsschritt 1 racemisches Bromnarwedin (III) mit Palladium (II) Acetat und Triphenylphosphin debromiert, in einem Reaktionsschritt 2 das Reaktionsgemisch, beinhaltend racemisches Narwedin (IV)' unter Sauerstoffkontakt aufarbeitet und in einem zum enantiomerenreinem Narwedin (V) umwandelt und wobei man in einem Reaktionsschritt 3 durch Reduktion enantiomerenreines Galanthamin der allgemeinen Formel (I) mit R₁ gleich CH₃ erhält und in einem Reaktionsschritt 4 durch Alkylierung bzw. Dealkylierung Verbindungen der allegemeinen Formel (I) bzw. in einem Reaktionsschritt 4' durch Alkylierung und Dealkylierung sowie anschließender Salzbildung Verbindungen der allgemeinen Formel (II) erhält.

Alternativ wird erfindungsgemäß ein Verfahren zur Herstellung der eingangs genannten Verbindungen mit der allgemeinen Formel (I) bzw. (II) vorgeschlagen, wobei man in einem Reaktionsschritt 1 racemisches Bromnarwedin (III) mit Palladium (II) Acetat und Triphenylphosphin debromiert, in einem Reaktionsschritt 2 das Reaktionsgemisch, beinhaltend racemisches Narwedin (IV) unter Verwendung von Peroxiden aufarbeitet und in einem zum enantiomerenreinem Narwedin (V) umwandelt und wobei man in einem Reaktionsschritt 3 durch Reduktion enantiomerenreines Galanthamin der allgemeinen Formel (I) mit R₁ gleich CH₃ erhält und in einem Reaktionsschritt 4 durch Alkylierung bzw. Dealkylierung Verbindungen der allgemeinen Formel (I) bzw. in einem Reaktionsschritt 4' durch Alyklierung und Dealkylierung sowie anschließender Salzbildung Verbindungen der allgemeinen Formel (II) erhält.

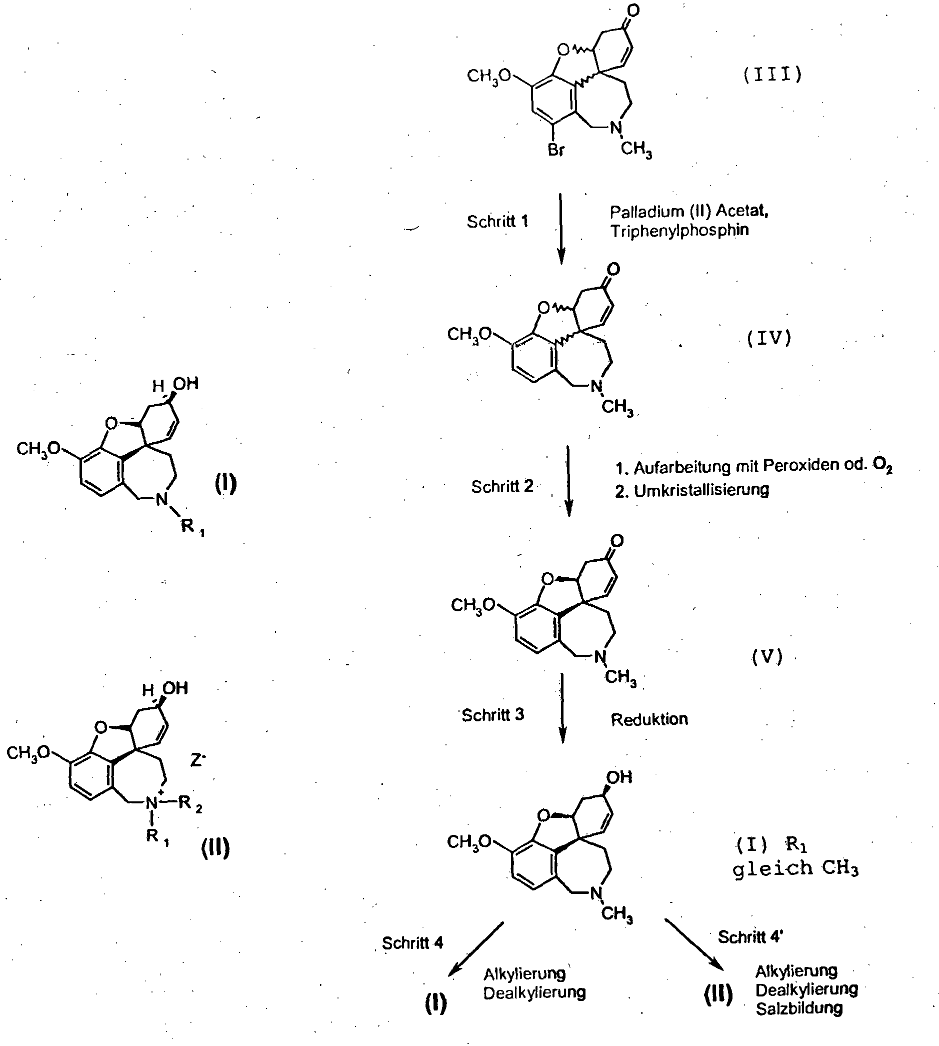

Vorteilhafte Ausgestaltungen der erfindungsgemäßen Verfahren sind gemäß Unteransprüche offenbart.

Die Erfindung betrifft weiters enantiomerenreines Narwedin der allgemeinen Formel (V), welches durch den Syntheseschritt 1 sowie den Syntheseschritt 2 hergestellt wird und einen Restanteil an Palladium von weniger als 26 ppm, bevorzugter Weise von weniger als 24 ppm, besonders bevorzugter Weise von weniger als 14 ppm aufweist.

Die Erfindung betrifft weiters enantiomerenreines Galanthamin der allgemeinen Formel (I) mit R₁ gleich CH₃, welches durch die Synthese-schritte 1 bis 3 mit einem oder mehreren jeweils nach geschalteten Reinigungsschritten, vorzugsweise Umkristallisieren, hergestellt wird und einen Restanteil an Palladium von weniger als 5 ppm aufweist.

Die Erfindung betrifft weiters Galanthaminderivate der allgemeinen Formeln (I) und (II), welche nach einem Verfahren mit den Syntheseschritten 1 bis 4 bzw. 4' sowie jeweils nachgeschalteten Reinigungsschritten, vorzugsweise Umkristallisieren, hergestellt werden und einen Restanteil an Palladium von weniger als 5 ppm aufweisen.

Die Erfindung betrifft weiters die Verwendung von enantiomerenreinem Galanthamin zur Herstellung von Arzneimitteln für die Behandlung neurodegenerativer Erkrankungen, wie die Alzheimer'sche Erkrankung.

Die Erfindung betrifft ebenso die Verwendung von hochreinen Galanthaminderivaten mit den allgemeinen Formeln (I) und (II) zur Herstellung von Arzneimitteln für die Behandlung neurodegenerativer Erkrankungen, wie die Alzheimer'sche Erkrankung.

Die Erfindung wird im folgenden anhand von Ausführungsbeispielen zur Durchführung der Erfindung näher erläutert, wobei auf die Verfahrensschritte gemäß Reaktionsschema Bezug genommen wird:

Schritt 1: Racemisches Bromnarwedin der allgemeinen Formel (III) wird in DMF aufgenommen, mit NaCO₂H, PPH₃, Palladium(II)Acetat sowie Natriumhydroxid versetzt. Diese Reaktionsmischung wird auf 94°C erhitzt und sechs Stunden auf dieser Temperatur gehalten, wobei der Reaktionsverlauf mittels Chromatographie verfolgt wird. Anschließend wird das Reaktionsgemisch aufgearbeitet, wobei man DMF abdestilliert, das racemische Narwedin (IV) durch Zugabe von Wasser ausfällt und abtrennt.

Schritt 2.1: Das erhaltene recemische Narwedin (IV) wird in einem Gemisch von Ethanol/Triethylamin aufgenommen und mit Aktivkohle und einem Filterhilfsmittel versetzt. Die Mischung wird unter intensivem Rühren ein bis vier Stunden unter Rückfluss erhitzt, wobei ein Luft-Stickstoffgemisch mit beispielsweise 5 Vol.% Sauerstoff durch den Reaktor geblasen wird. Überraschender Weise wurde gefunden, dass durch die Behandlung mit Aktivkohle einerseits und den Sauerstoffkontakt anderseits die Reduktion der Palladiumanteile von deutlich über 95 % im Vergleich zu bekannten, nachweisbaren Palladiumanteilen erreicht werden konnte. Dies soll anhand folgender Tabelle näher erläutert werden:

| | 1. Charge | 2. Charge | 3. Charge |
|---|---|---|---|
| | Pd (ppm) | Pd (ppm) | Pd (ppm) |
| Racemisches Narwedin | 813 | 748 | 753 |
| (-)-Narwedin | 24 | 26 | 14 |

Aus dieser tabellarischen Aufstellung ist zu ersehen, dass im racemischen Narwedingemisch Palladiumreste von 748 bis 813 ppM nachweisbar sind. Reaktionsendprodukte mit diesen Anteilen an Palladium sind für eine weitere Verwendung für die Herstellung eines Arzneimittels ungeeignet. Durch die erfindungsgemäße Aufarbeitung des Reaktionsgemisches mit Aktivkohle bei gleichzeitigem Sauerstoffkontakt wird der Palladiumkatalysator in eine unlösliche, oxidierte Form übergeführt, sodass eine Abtrennung in einem ppm-Bereich von weniger als 26, bevorzugter Weise von weniger als 24, besonders bevorzugter Weise von weniger als 14 möglich ist.

In einer alternativen Verfahrensvariante wird das erhaltene racemische Narwedin (IV) ebenso in einem Gemisch von Ethanol/Triethylamin aufgenommen und mit Aktivkohle und einem Filtrierhilfsmittel versetzt; allerdings wird diese Mischung anschließend unter intensivem Rühren mit 0,1 -1 Gew% Wasserstoffperoxid langsam versetzt und ein bis vier Stunden unter Rückfluss erhitzt. Überraschender Weise wurde auch bei dieser Verfahrensvariante gefunden, dass durch die Behandlung mit Aktivkohle einerseits und die Verwendung von Wasserstoffperoxid andererseits nach Filtration der Palladiumanteil im Vergleich zu bekannten, nachweisbaren Palladiumanteilen deutlich reduziert werden konnte. Die gemessenen Werte sind der folgenden Tabelle zu entnehmen:

| | **1.Charge** | **2.Charge** | **3.Charge** |
|---|---|---|---|
| | **Pd (ppm)** | **Pd (pmm)** | **Pd (ppm)** |
| **Racemisches Narwedine** | 800 | 810 | 763 |
| **(-)-Narwedine (H2O2 behandelt)** | 22 | 24 | 16 |

In einer weiteren Verfahrensvariante wird die Mischung bestehend aus racemischem Narwedin (IV), Ethanol, Triethylamin, Aktivkohle und einem Filtrierhilfsmittel unter intensivem Rühren mit 0,1 -1 Gew% Metachlorperbenzosäure versetzt und ein bis vier Stunden unter Rückfluss erhitzt. Auch bei dieser Verfahrensvariante wurde Überraschender Weise gefunden, dass durch die Behandlung mit Aktivkohle einerseits und die Verwendung von Metachlorperbenzosäure andererseits nach Filtration der Palladiumanteil im Vergleich zu bekannten, nachweisbaren Palladiumanteilen wesentlich reduziert werden konnte. Die ermittelten Werte sind in folgender Tabelle angeführt:

| | **1.Charge** | **2.Charge** | **3.Charge** |
|---|---|---|---|
| | **Pd (ppm)** | **Pd (ppm)** | **Pd (ppm)** |
| **Racemisches Narwedine** | 778 | 805 | 767 |
| **(-)-Narwedine (MCPBA behandelt)** | 20 | 23 | 18 |

Schritt 2.2: Das nach Schritt 2.1 erhaltene Reaktionsgemisch wird gekühlt und mit(-)Narwedinkristallen angeimpft, sodass enantiomeren-reines (-)Narwedin mit der allgemeinen Formel (V) erhalten wird. Schritt 3: Das nach dem Umkristallisieren erhaltene enantiomeren-reine (-)Narwedin mit der allgemeinen Formel (V) wird, wie in der WO-A-96/12692 beschrieben, mit einer einmolaren Lösung von L-Selektrid in THF versetzt, eine Stunde rühren gelassen, mit Ethanol versetzt und eingedampft. Durch die enantiomerselektive Reduktion wird enantiomerenreines Galanthamin der allgemeinen Formel (I) für R₁=CH₃ erhalten. Durch ein- oder mehrmaliges Umkristallisieren werden Restanteil von Palladium von weniger als 5 ppm erzielt. Dies deshalb, da durch die Aufarbeitung mit Sauerstoff oder Peroxid gemäß Syntheseschritt 2.1 der Palladiumkatalysator in eine unlösliche, oxidierte Form übergeführt wird, welche sich im Zuge der Reinigung durch Umkristallisieren leicht abtrennen lässt.

Schritt 4: Die Verbindung der allgemeinen Formel (I) mit R₁ gleich CH₃ kann einer Dealkylierung bzw. einer weiteren Alkylierung unterworfen werden, um so die Reste R₁ bzw., R₂ am Stickstoff-Atom einzuführen.

Schritt4': Schritt 4' erfolgt analog zu Schritt 4 mit dem Unterschied, dass eine weitere Umsetzung mit einer Säure, wie beispielsweise Hydrobromid, zu pharmazeutisch akzeptablen Salzen mit Gegenanionen Z⁻ wie beispielsweise ein Bromid, erfolgt. Auch die Verbindungen mit der allgemeinen Formel (I) oder (II) können, falls notwendig, weiter durch Umkristallisieren gereinigt werden, sodass ein Restanteil von weniger als 5 ppm erzielt wird.

Die vorgenannten Ausführungsbeispiele wurden derart durchgeführte, dass R₁ bzw. R₂ ein Substituentenmuster zeigen, worin aliphatische Kohlenstoffsubstituenten eine Anzahl von Kohlenstoffen im Bereich von 1 bis 6 aufweisen, und der aromatische Rest Aryl aus der Gruppe Furyl, Phenyl, Pyridinyl, Pyridazyl, Pyrazinyl, Pyrazolyl, Imidazyl und Pyrazyl ausgewählt wird.

Die pharmakologische Wirkung der Verbindungen gemäß der allgemeinen Formeln (I) und (II) lässt sich anhand der gemessenen IC⁵⁰ Werte belegen, da diese jene Konzentrationen repräsentieren, bei welchen eine 50%-ige Hemmung der Acetylcholinesterase (AChEI) bzw. der Butyrylcholinesterase (BuCHEI) eintritt. Zufriedenstellende Hemmwerte - siehe folgende Übersicht - sind des Weiteren ein Indiz dafür, dass die Verbindungen der allgemeinen Formeln (I) bzw. (II) zur Herstellung von Arzneimitteln für die Behandlung von neurodegenerativen Erkrankungen, wie die Alzheimer'sche Erkrankung, geeignet sind.

| BEISPIEL | FORMELBILD | FORSCHUNGS-CODE | IC50 AChEI | IC50 BuChEI |
|---|---|---|---|---|
| 1 | 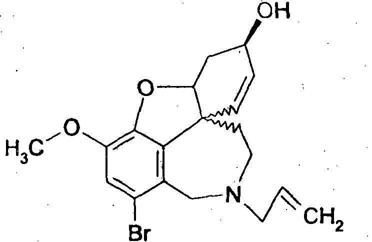 | SPH-1097 | 200 | 2.6 |
| 2 | 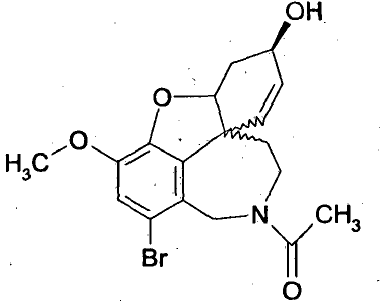 | SPH-1071 | 49 | 14 |
| 3 | 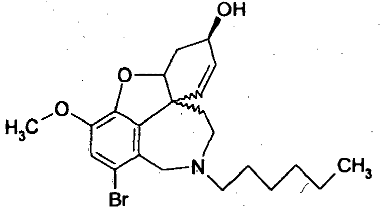 | SPH-1054 | 12 | 0.2 |
| 4 | 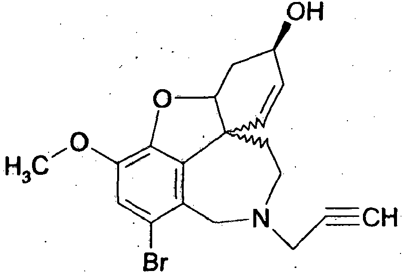 | SPH-1075 | 63 | 10 |
| 5 | 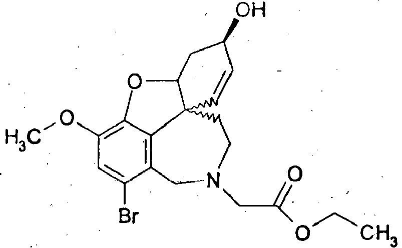 | SPH-1080 | 200 | 56 |
| 6 | 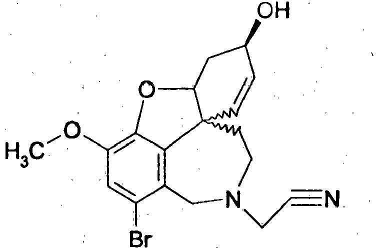 | SPH-1069 | 200 | 70 |
| 7 | 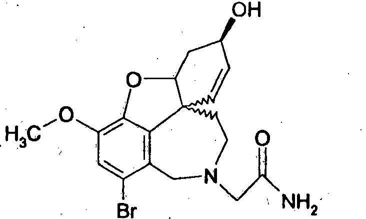 | SPH-1081 | 200 | 15 |
| 8 | 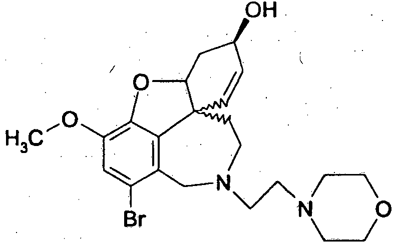 | SPH-1078 | 200 | 4.4 |
| 9 | 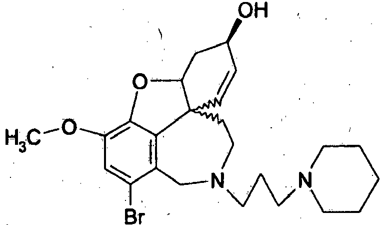 | SPH-1106 | 23 | 3.3 |
| 10 | 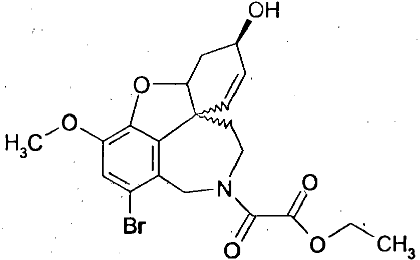 | SPH-1070 | 32.5 | 11 |
| 11 | 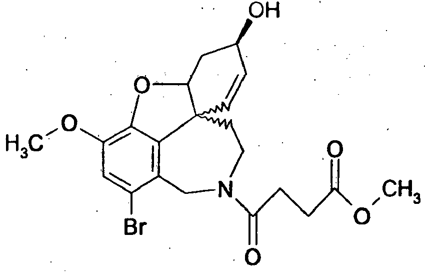 | SPH-1072 | 200 | 200 |
| 12 | 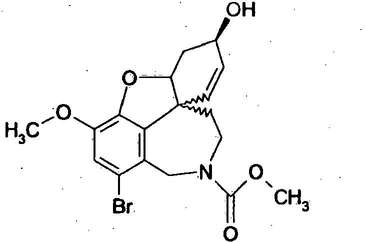 | SPH-1082 | 200 | 200 |
| 13 | 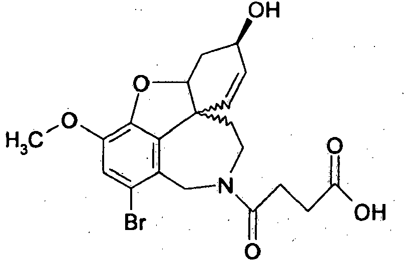 | SPH-1090 | 200 | 200 |
| 14 | 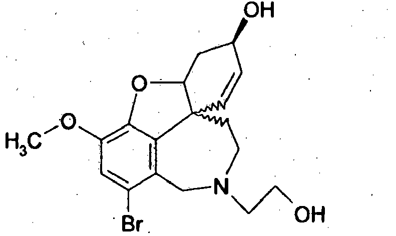 | SPH-1095 | 34 | 6.35 |
| 15 | 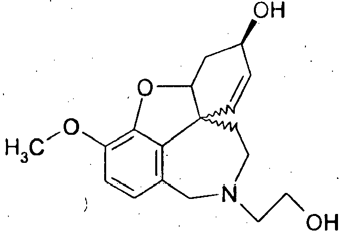 | SPH-1096 | 15 | 19 |
| 16 | 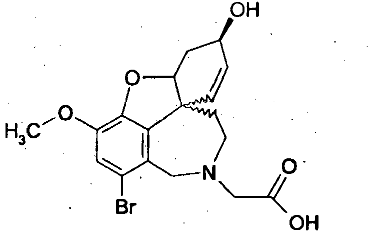 | SPH-1089 | 200 | 200 |
| 17 | 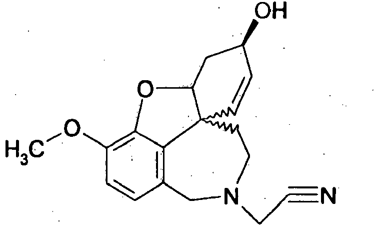 | SPH-1099 | 37 | 200 |
| 18 | 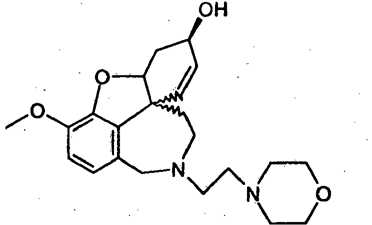 | SPH-1098 | 13 | 7 |
| 19 | 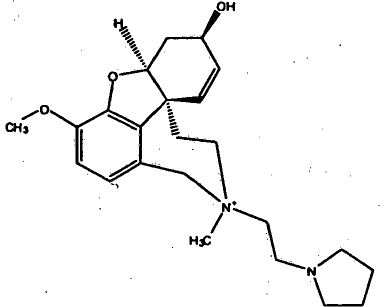 | SPH-1023 | 60 | 8 |
| 20 | 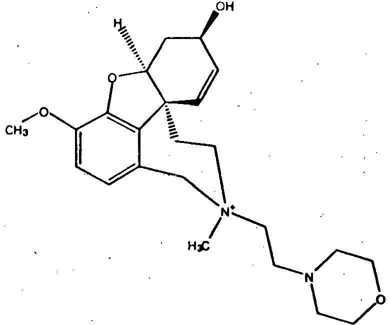 | SPH-1098 (rac) | 13 (rac) | 7 (rac) |
| 21 | 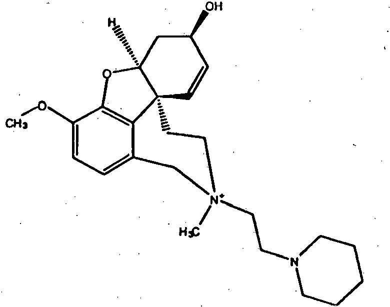 | SPH-1144 | 6,2 | 3,6 |
| 22 | 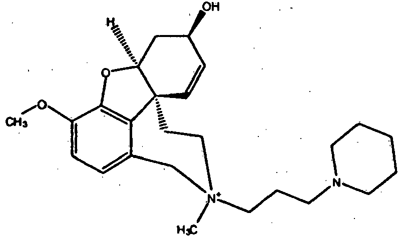 | SPH-1019 | 30 | 5,6 |
| 23 | 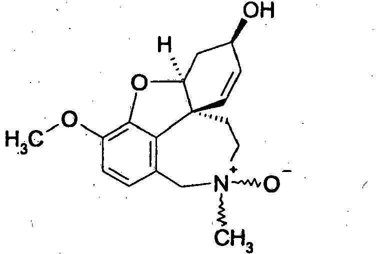 | SPH-1052 | 200 | 200 |
| 24 | 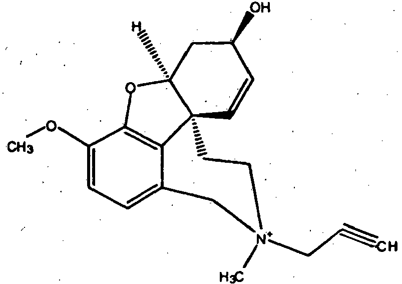 | SPH-1058 | 1,35 | 1,6 |
| 25 | 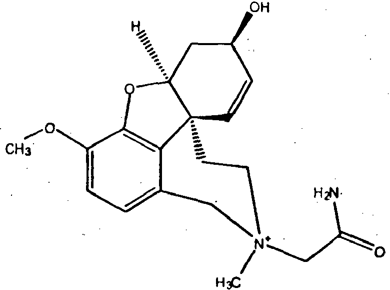 | SPH-1140 | 3,1 | 2,5 |
| 26 | 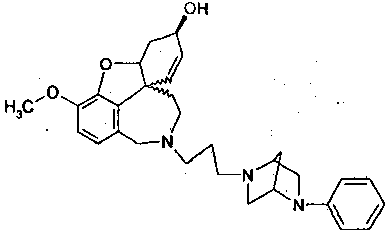 | SPH-1195 | 24.5 | 7.5 |
| 27 | 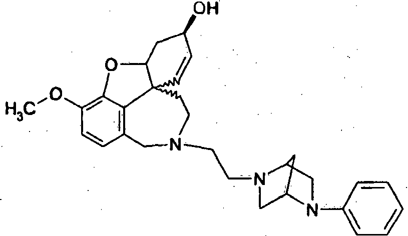 | SPH-1329 | 2.9 | 0.9 |

Zusammenfassend kann gesagt werden, dass durch die erfindungsgemäße Aufarbeitung eines durch Palladiumkatalyse gewonnenen debromierten Narwedin, nämlich durch Kontakt mit Sauerstoff oder Peroxiden, der eingesetzte Palladiumkatalysator in eine unlösliche Oxidform übergeführt und in einfacher Weise abgetrennt werden kann. Durch diese den Sicherheitsvorschriften durchaus gerecht werdende Aufarbeitung des Reaktionsgemisches gelang es in überraschender Weise die Palladiumreste unterhalb von 5 ppm zu reduzieren, sodass hochreines Galanthamin bzw. hochreine Galanthaminderivate gewonnen werden konnten, welche(s) unmittelbar in die Herstellung von Arzneimitteln, wie beispielsweise solche für die Behandlung der Alzheimer'schen Erkrankung, eingesetzt werden konnten (kann).

## Patentansprüche

1. Verfahren zum Herstellen von hochreinem 4a,5,9,10,11,12,-Hexahydro-6H-benzofuro[3a,3,2-ef][2]benzazepin sowie dessen Derivaten mit der allgemeinen Formel I und II 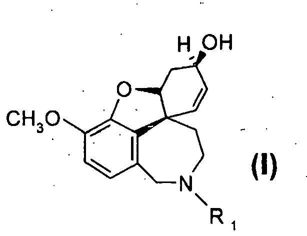 und 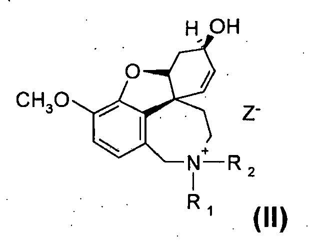 oder Salzen derselben, worin R₁ ausgewählt ist aus der Gruppe bestehend aus Wasserstoff, Hydroxy, Alkoxy, (C₁-C₆) Alkyl, welches gegebenenfalls durch wenigstens ein Halogen substituiert ist, (C₁-C₆) Alkenyl, (C₁-C₆) Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und worin R₂ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl und wobei Z⁻ ein Anion einer pharmazeutisch annehmbaren organischen Säure oder ein anorganisches Anion ist, **dadurch gekennzeichnet, dass** man in einem Reaktionsschritt 1 racemisches Bromnarwedin (III) 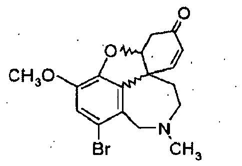 mit Palladium(II)Acetat und Triphenylphosphin debromiert, in einem Reaktionsschritt 2 das Reaktionsgemisch, beinhaltend racemisches Narwedin (IV) 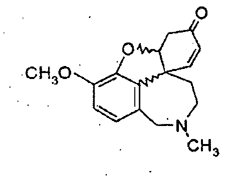 unter Sauerstoffkontakt aufarbeitet und in einem zum enantiomerenreinem Narwedin (V) 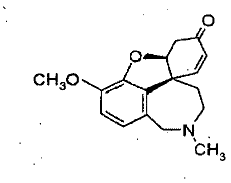 umwandelt, wobei ein Luft-Stickstoffgemisch, das 0,2 bis 20 Vol% Sauerstoff enthält, in Gegenwart von Aktivkohle durch den Reaktor geblasen wird, und wobei man in einem Reaktionsschritt 3 durch Reduktion enantiomerenreines Galanthamin der allgemeinen Formel (I) mit R₁ gleich CH₃ erhält und in einem Reaktionsschritt 4 durch Alkylierung bzw. Dealkylierung Verbindungen der allgemeinen Formel (I) bzw. in einem Reaktionsschritt 4' durch Alkylierung und Dealkylierung sowie anschließender Salzbildung Verbindungen der allgemeinen Formel (II) erhält.

2. Verfahren nach Anspruch 1, **dadurch gekennzeichnet, dass** dem Reaktionsschritt 3 und/ oder dem Reaktionsschritt 4 ein oder mehrere Reinigungsschritt(e), welche durch Umkristallisieren erfolgen, nachgeschaltet sind (ist).

3. Verfahren zum Herstellen von hochreinem 4a,5,9,10,11,12,-Hexahydro-6H-benzofuro[3a,3,2-ef][2]benzazepin sowie dessen Derivaten mit der allgemeinen Formel I und II 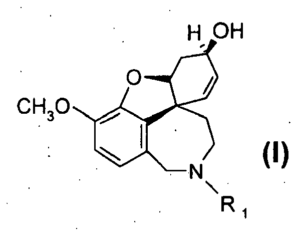 und 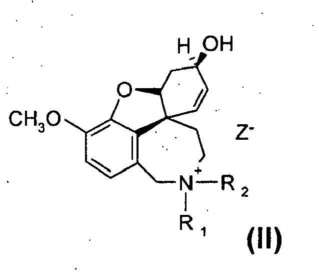 oder Salzen derselben, worin R₁ ausgewählt ist aus der Gruppe bestehend aus Wasserstoff, Hydroxy, Alkoxy, (C₁-C₆) Alkyl, welches gegebenenfalls durch wenigstens ein Halogen substituiert ist, (C₁-C₆) Alkenyl, (C₁-C₆) Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und worin R₂ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl und wobei Z⁻ ein Anion einer pharmazeutisch annehmbaren organischen Säure oder ein anorganisches Anion ist, **dadurch gekennzeichnet, dass** man in einem Reaktionsschritt 1 racemisches Bromnarwedin (III) 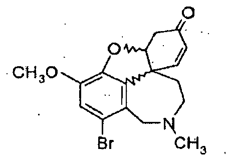 mit Palladium(II)Acetat und Triphenylphosphin debromiert, in einem Reaktionsschritt 2 das Reaktionsgemisch, beinhaltend racemisches Narwedin (IV) 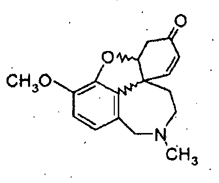 in Gegenwart von Peroxiden und Aktivkohle aufarbeitet und in einem zum enantiomerenreinem Narwedin (V) 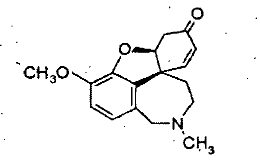 umwandelt und wobei man in einem Reaktionsschritt 3 durch Reduktion enantiomerenreines Galanthamin der allgemeinen Formel (I) mit R₁ gleich CH₃ erhält und in einem Reaktionsschritt 4 durch Alkylierung bzw. Dealkylierung Verbindungen der allgemeinen Formel (I) bzw. in einem Reaktionsschritt 4' durch Alkylierung und Dealkylierung sowie anschließender Salzbildung Verbindungen der allgemeinen Formel (II) erhält.

4. Verfahren nach Anspruch 3, **dadurch gekennzeichnet, dass** im Reaktionsschritt 2 als Peroxide anorganische Peroxide, eingesetzt werden.

5. Verfahren nach Anspruch 4, **dadurch gekennzeichnet, dass** als anorganisches Peroxid Wasserstoffperoxid eingesetzt wird.

6. Verfahren nach Anspruch 3, **dadurch gekennzeichnet, dass** im Reaktionsschritt 2 als Peroxide organische Peroxide eingesetzt werden.

7. Verfahren nach Anspruch 6, **dadurch gekennzeichnet, dass** als organisches Peroxid Metachlorperbenzoesäure eingesetzt wird.

8. Verfahren nach einem der Ansprüche 3 bis 7, **dadurch gekennzeichnet, dass** dem Reaktionsschritt 3 und/ oder dem Reaktionsschritt 4 ein oder mehrere Reinigungsschritt(e), welche durch Umkristallisieren erfolgen, nachgeschaltet sind (ist).

## Claims

1. Process for the production of high-purity 4a,5,9,10,11,12,-hexahydro-6H-benzofuro[3a,3,2-ef][2] benzazepine and derivatives thereof with the general formulas I and II 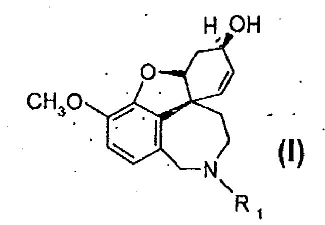 and 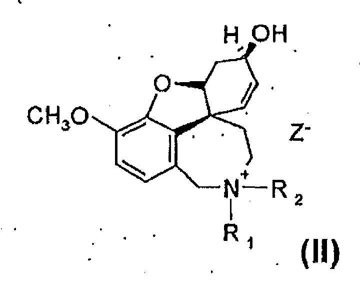 or salts thereof, in which R₁ is selected from the group that consists of hydrogen, hydroxy, alkoxy, (C₁-C₆) alkyl, which optionally is substituted by at least one halogen, (C₁-C₆) alkenyl, (C₁-C₆) alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl, arylsulfonyl, and in which R₂ is selected from the group that consists of hydrogen, formyl, alkyl, alkenyl, aryl, aralkyl, alkylcarbonyl, arylcarbonyl, aralkylcarbonyl, alkyloxycarbonyl, aryloxycarbonyl, aralkyloxycarbonyl, alkylsulfonyl, arylsulfonyl and aralkylsulfonyl, and wherein Z⁻ is an anion of a pharmaceutically acceptable organic acid or an inorganic anion, **characterised in that**, in a reaction step 1, racemic bromine narwedine (III) 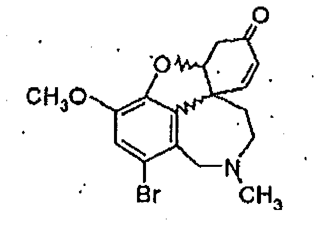 is debrominated with palladium (II) acetate and triphenylphosphine, in a reaction step 2, the reaction mixture that contains racemic narwedine (IV) 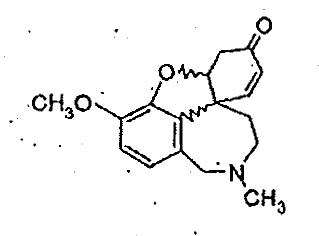 is worked up under oxygen contact and converted into an enantiomerically pure narwedine (V) 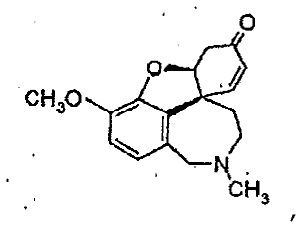 wherein an air/nitrogen mixture that contains 0.2 to 20 vol. % of oxygen is blown through the reactor in the presence of activated carbon, and wherein, in a reaction step 3, enantiomerically pure galanthamine of general formula (I) with R₁ equal to CH₃ is obtained by reduction, and, in a reaction step 4, compounds of general formula (I) are obtained by alkylation or dealkylation, or, in a reaction step 4', compounds of general formula (II) are obtained by alkylation and dealkylation as well as subsequent salt formation.

2. The process according to Claim 1, **characterised in that** one or more purification step(s), which occur by recrystallisation, is/are downstream to the reaction step 3 and/or the reaction step 4.

3. A process for the production of high-purity 4a,5,9,10,11,12,-hexahydro-6H-benzofuro[3a,3,2-ef][2] benzazepine and derivatives thereof with the general formulas I and II 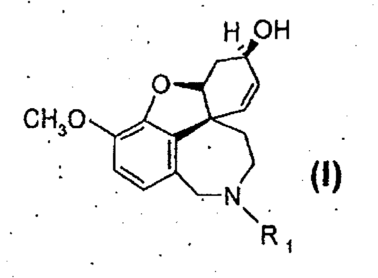 and 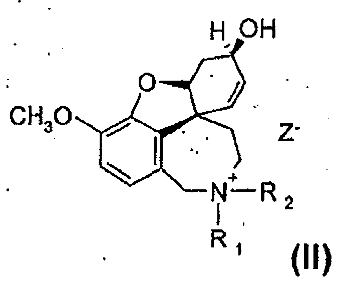 or salts thereof, in which R₁ is selected from the group that consists of hydrogen, hydroxy, alkoxy, (C₁-C₆) alkyl, which optionally is substituted by at least one halogen, (C₁-C₆) alkenyl, (C₁-C₆) alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl, arylsulfonyl, and in which R₂ is selected from the group that consists of hydrogen, formyl, alkyl, alkenyl, aryl, aralkyl, alkylcarbonyl, arylcarbonyl, aralkylcarbonyl, alkyloxycarbonyl, aryloxycarbonyl, aralkyloxycarbonyl, alkylsulfonyl, arylsulfonyl and aralkylsulfonyl, and wherein Z-is an anion of a pharmaceutically acceptable organic acid or an inorganic anion, **characterised in that**, in a reaction step 1, racemic bromine narwedine (III) 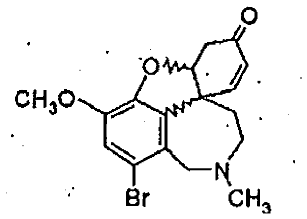 is debrominated with palladium (II) acetate and triphenylphosphine, in a reaction step 2, the reaction mixture that contains racemic narwedine (IV) 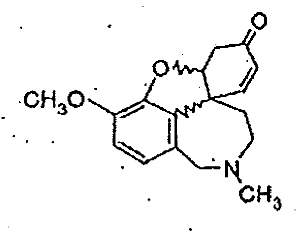 is worked up in the presence of peroxides and activated carbon and converted into an enantiomerically pure narwedine (V) 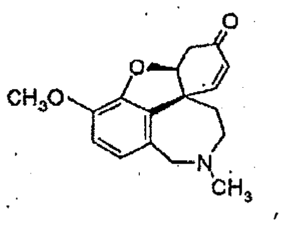 and wherein, in a reaction step 3, enantiomerically pure galanthamine of general formula (I) with R₁ equal to CH₃ is obtained by reduction, and, in a reaction step 4, compounds of general formula (I) are obtained by alkylation or dealkylation, or, in a reaction step 4', compounds of general formula (II) are obtained by alkylation and dealkylation as well as subsequent salt formation.

4. The process according to Claim 3, **characterised in that**, in reaction step 2, inorganic peroxides are used as peroxides.

5. The process according to Claim 4, **characterised in that** hydrogen peroxide is used as inorganic peroxide.

6. The process according to Claim 3, **characterised in that**, in the reaction step 2, organic peroxides are used as peroxides.

7. The process according to Claim 6, **characterised in that** metachloroperbenzoic acid is used as organic peroxide.

8. The process according to one of Claims 3 to 7, **characterised in that** one or more purification step(s), which occur by recrystallisation, is/are downstream to the reaction step 3 and/or the reaction step 4.

## Revendications

1. Procédé de préparation de 4a,5,9,10,11,12,-hexahydro-6H-benzofuro[3a,3,2-ef][2]benzazépine de haute pureté ainsi que de ses dérivés répondant à la formule générale I et II 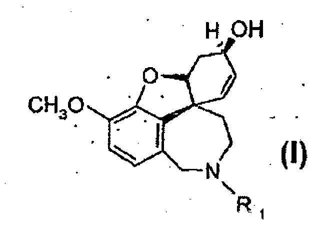 et 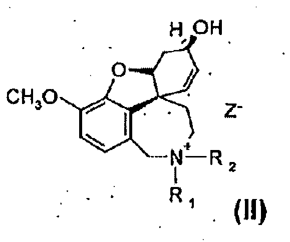 ou de leurs sels, R₁ étant choisi dans le groupe constitué par hydrogène, hydroxyle, alkoxy, alkyle en C₁-C₆ pouvant le cas échéant présenter au moins un halogène en tant que substituants, alcènyle en C₁-C₆, alcynyle en C₁-C₆, aryle, aralkyle, aryloxyalkyle, formyle, alkylcarbonyle, arylcarbonyle, aralkylcarbonyle, alkyloxycarbonyle, aryloxycarbonyle, aralkyloxycarbonyle, alkylsulfonyle, aralkylsulfonyle, arylsulfonyle, et R₂ étant choisi dans le groupe constitué par hydrogène, formyle, alkyle, alcènyle, aryle, aralkyle, alkylcarbonyle, arylcarbonyle, aralkylcarbonyle, alkyloxycarbonyle, aryloxycarbonyle, aralkyloxycarbonyle, alkylsulfonyle, arylsulfonyle et aralkylsulfonyle et Z- étant un anion d'un acide organique pharmaceutiquement acceptable ou un anion inorganique, **caractérisé en ce que**, dans une étape de réaction 1, on réalise la débromation de la bromonarwédine racémique (III) 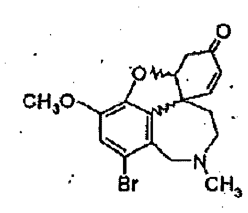 en mettant en oeuvre de l'acétate de palladium(II) et de la triphénylphosphine, pour soumettre, dans une étape de réaction 2, le mélange réactionnel contenant de la narwédine racémique (IV) 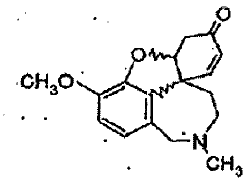 à un traitement ultérieur réalisé au contact avec l'oxygène, puis le convertir en narwédine (V) sous forme énantiomérique pure 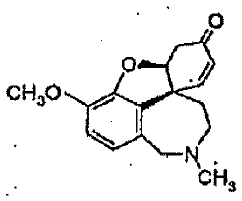 en introduisant dans le réacteur, en présence de charbon actif, un mélange air/azote contenant 0,2 à 20 % en volume d'oxygène, et pour obtenir, dans une étape de réaction 3 consistant à réaliser une réduction, une forme énantiomérique pure de la galanthamine répondant à la formule générale (I), R₁ étant CH₃, et pour obtenir, dans une étape de réaction 4 consistant à réaliser une alkylation ou bien une désalkylation, des composés répondant à la formule générale (I) ou bien, dans une étape de réaction 4' consistant à réaliser une alkylation et une désalkylation puis à former un sel, des composés répondant à la formule générale (II).

2. Procédé selon la revendication 1, **caractérisé en ce qu'**en aval de l'étape de réaction 3 et/ou de l'étape de réaction 4, on met en oeuvre une ou plusieurs étape(s) de réaction consistant à réaliser une recristallisation.

3. Procédé de préparation de 4a,5,9,10,11,12,-hexahydro-6H-benzofuro[3a,3,2-ef][2]benzazépine de haute pureté ainsi que de ses dérivés répondant à la formule générale I et II 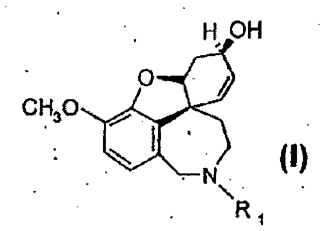 et 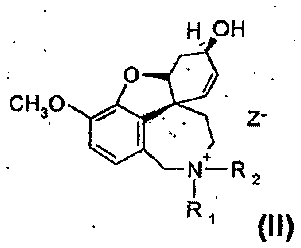 ou de leurs sels, R₁ étant choisi dans le groupe constitué par hydrogène, hydroxyle, alkoxy, alkyle en C₁-C₆ pouvant le cas échéant présenter au moins un halogène en tant que substituants, alcènyle en C₁-C₆, alcynyle en C₁-C₆, aryle, aralkyle, aryloxyalkyle, formyle, alkylcarbonyle, arylcarbonyle, aralkylcarbonyle, alkyloxycarbonyle, aryloxycarbonyle, aralkyloxycarbonyle, alkylsulfonyle, aralkylsulfonyle, arylsulfonyle, et R₂ étant choisi dans le groupe constitué par hydrogène, formyle, alkyle, alcènyle, aryle, aralkyle, alkylcarbonyle, arylcarbonyle, aralkylcarbonyle, alkyloxycarbonyle, aryloxycarbonyle, aralkyloxycarbonyle, alkylsulfonyle, arylsulfonyle et aralkylsulfonyle et Z⁻ étant un anion d'un acide organique pharmaceutiquement acceptable ou un anion inorganique, **caractérisé en ce que**, dans une étape de réaction 1, on réalise la débromation de la bromonarwédine racémique (III) 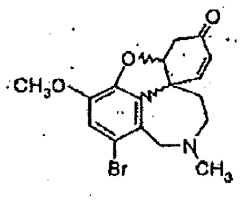 en mettant en oeuvre de l'acétate de palladium(II) et de la triphénylphosphine, pour soumettre, dans une étape de réaction 2, le mélange réactionnel contenant de la narwédine racémique (IV) 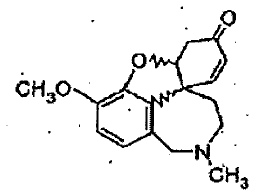 à un traitement ultérieur réalisé en présence de peroxydes et de charbon actif, puis le convertir en narwédine (V) sous forme énantiomérique pure 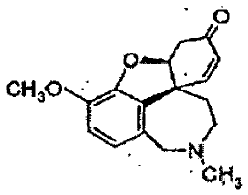 et pour obtenir, dans une étape de réaction 3 consistant à réaliser une réduction, une forme énantiomérique pure de la galanthamine répondant à la formule générale (I), R₁ étant CH₃, et pour obtenir, dans une étape de réaction 4 consistant à réaliser une alkylation ou bien une désalkylation, des composés répondant à la formule générale (I) ou bien, dans une étape de réaction 4' consistant à réaliser une alkylation et une désalkylation puis à former un sel, des composés répondant à la formule générale (II).

4. Procédé selon la revendication 3, **caractérisé en ce que** dans l'étape de réaction 2, on met en oeuvre des peroxydes, inorganiques à titre de peroxydes.

5. Procédé selon la revendication 4, **caractérisé en ce que** l'on met en oeuvre du peroxyde d'hydrogène à titre de peroxyde inorganique.

6. Procédé selon la revendication 3, **caractérisé en ce que** dans l'étape de réaction 2, on met en oeuvre des peroxydes organiques à titre de peroxydes.

7. Procédé selon la revendication 6, **caractérisé en ce que** l'on met en oeuvre de l'acide métachlorobenzoïque à titre de peroxyde organique.

8. Procédé selon l'une des revendications 3 à 7, **caractérisé en ce que** l'on met en oeuvre, en aval de l'étape de réaction 3 et/ou de l'étape de réaction 4, une ou plusieurs étapes de purification consistant à réaliser une recristallisation.
